# EUROPEAN PATENT SPECIFICATION

(11) **EP 2 726 144 B1**
(45) Date of publication and mention of the grant of the patent: **10.02.2016**
(21) Application number: 12748257.8
(22) Date of filing: 29.06.2012
(51) Int. Cl.: A61N 1/08, A61N 1/05, A61B 5/00, A61B 5/053, A61B 5/06

(54) **ARRANGEMENT FOR DEFINING A LOCATION WITHIN AN ORGANISM AND METHOD FOR MANUFACTURING A MANDRIN TO BE ACCOMMODATED IN A NEEDLE**
VORRICHTUNG ZUR FESTLEGUNG EINER STELLE IN EINEM ORGANISMUS UND VERFAHREN ZUR HERSTELLUNG EINES MANDRINS IN EINER NADEL
DISPOSITIF POUR DÉFINIR UN SITE À L'INTÉRIEUR D'UN ORGANISME ET PROCÉDÉ POUR FABRIQUER UN MANDRIN À LOGER DANS UNE AIGUILLE

(30) Priority: 30.06.2011 FI 20115684
(43) Date of publication of application: 07.05.2014
(73) Proprietor: Injeq OY, 33720 Tampere (FI)
(72) Inventor: KRONSTRÖM, Kai, 02200 Espoo (FI); KONTIOLA, Antti, 00600 Helsinki (FI); PAASSILTA, Katja, 23200 Kangasala (FI); AHONEN, Petri, 33720 Tampere (FI); HYTTINEN, Jari, 33900 Tampere (FI)
(74) Representative: Väisänen, Olli Jaakko
(86) International application number: PCT/IB2012/053333
(87) International publication number: WO 2013/001510

(56) References cited:
- US-A1- 2004 010 204
- US-A1- 2010 256 483
- US-B1- 6 337 994

## Description

### Field of the invention

The invention is more generally related to the field of medical technology. The invention is specifically related to needles and needle points as well as to devices and methods for monitoring the advancement of the above penetrating a tissue.

### Technical background

Many therapeutic, surgical, diagnostic and cosmetic interventions involve doses of drugs or substances related to the intervention, e.g. markers, being administered to a patient's organism. Said substances are usually in the term of liquid or gas and they are conveyed into the tissue through a hollow needle. Medical interventions are also known wherein substances are removed from the organism through a hollow needle or wherein a specimen is taken of a tissue by using a needle.

It is essential in all above-mentioned interventions that the needle point be led into the right tissue and that it is Known when the point is in the right tissue. For example in anesthesia of the nerves in the spinal cord, i.e. in spinal or in epidural anesthesia, severe complications such as unconsciousness or even death, may result if the local anesthetic is being administered to the wrong tissue.

Systems for defining the location of a needle, based on bioimpedance measurements, are known. One of these is presented in publication US 6,337,994. The problem with this is, however, that the interrelationship between the needle and the mandrin defines the mutual location of electrodes. It is thus not possible, for example, to focus the measurement range on a smaller ares than the one allowed by the geometries of the needle and the mandrin.

### Objective of the invention

The invention aims at enabling a more precise definition of the location of a needle in an organism, especially in order to monitor the advancement of the needle or the needle point penetrating a tissue.

This aim can be achieved by means of the arrangement for defining a location in an organism according to claim 1 as well as by means of the method for manufacturing a mandarin to be accommodated in a needle in accordance with claim 11.

The dependent claims describe advantageous embodiments of the arrangement and the method.

### Advantages of the invention

With the arrangement for defining a location in an organism comprising
i) a hollow needle, consisting of a longitudinal channel and a distil end;
ii) a mandrin, accommodated in the longitudinal channel in mobile manner in relation to it and removable from said channel, having a distal end of closed design comprising an electrode pattern including at least two electrodes; as well as
iii) a processing unit, configured to monitor the advancement of the distal end only on the basis of a measurement signal of bioimpedance received from the electrodes of the electrode pattern,
all wires leading to the measuring devices can be disposed of to prevent them from disturbing the useability when the mandrin is removed from the channel of the needle after positioning the needle, since the distal end of the mandrin comprises all the electrodes measuring the bioimpedance necessary for monitoring the advancement of the distale end of the needle.

With the functional electrode pattern in the arrangement comprising two voltage electrodes and two current electrodes, constant current can be led through the first two electrodes enabling to measure the voltage difference from the third and the fourth electrode, whereby the measurement is typically extremely free of interferences and accurate.

With the distal end of the mandarin in the arrangement comprising a sharp point, and especially with this sharp point being formed by a bevel edge, and with the electrodes being accommodated on said bevel, the contact between the electrodes and a tissue is improved by the tissue being pressed against said bevel when the needle is being pushed into the tissue. The bevel is preferably a plane surface which is an advantageous form to manufacture. The beval comprises a ridge accommodating at least one electrode, whereby the contact with the tissue can be improved.

With the electrode pattern comprising two pairs of electrodes, each with a current electrode and a voltage electrode, the distance between these pairs of electrodes being greater than the distance between the electrodes belonging to one and the same pair of electrodes, and with the electrodes covering at least 20% of the surface area of the distal end of the mandrin available, it is possible to reduce the risk of a malfunction of the electrodes, for example due to air bubbles.

With the needle being a flexible catheter which can be bent according to a canal of the organism, the arrangement enabling to accurately define a location can be used for conveying liquids to the organism or from the organism.

With at least some of the electrodes being formed by means of at least one metal wire by cutting or by breaking it off and optionally also by coating its end that is to become an electrode, whereby the metal wire is at the same time useable as a conductor from the electrode toward the processing unit, a possibly challenging connecting of a separate electrode end can be avoided or in any case made farther away from the distal end.

Avoiding the separate connecting is advantageous in the sense that possible contact faults, possibly having fatal consequences for the patient on whom the arrangement is used, can be better avoided or even eliminated.

With at least some electrodes of the electrode pattern being arranged coaxially, the distal end of the mandrin forms a kind of coaxial cable, whereby the signal traversing through the distal end can be better protected from electromagnetic interferences.

With the body of the mandrin being exposed in such a way that it is useable as one of the electrodes, the body of the mandrin can also be utilized as one of the electrodes.

In the method for manufacturing a mandrin to be accommodated in a needle, an electrode pattern is accommodated at the closed distal end of the mandrin, containing at least two electrodes that are suitable for measuring the bioimpedance of tissues and that are useable for generating a measurement signal of bioimpedance within an organism, on the basis of which the advancement of the distal end can be monitored by means of a processing unit. The advantage of the method lies in the fact that it is thus possible to define a location of a needle within an organism more accurately since all electrodes to be used for defining the location are located at the closed distal end of the mandrin and any measurement interferences due to the distance between the needle and the mandrin car thus be better avoided.

With the electrode being made by printing or pressing an electrically conductive functional electrode pattern on a nonconductive foil, followed by a conductor being accommodated in said electrode pattern and the mandrin being formed by arranging a functional electrode pattern at its distal end and the conductor being arranged in the body of the mandrin, it is possible to make the required fairly small electrode pattern accurately even on an industrial scale.

If said conductor is printed or pressed on said foil, followed by the foil being bent and the body of the mandrin being cast around the foil in such a way that the functional electrode pattern is left on the surface of the distal end of the mandrin and the conductor is left inside the body of the mandrin, it is possible to secure the electrode pattern in its place better due to casting. We are thus better able to manage the variations that occur in series production between the mandrins manufactured.

With the electrically conductive functional electrode pattern being printed or pressed on said substrate, said substrate being in the form of plane surface, it is possible to carry out the printing or pressing process in a more simple manner and more cheaply compared to carrying out the printing or pressing process for example on a bent foil since we are thus able to use traditional and commercially available printing and pressing devices for manufacturing the mandrin.

With the manufacturing method comprising the manufacturing of the body of the mandrin, as well as the printing or pressing of the electrodes and/or the printing or pressing of the related conductors on the surface of the body, the stages of manufacturing operation can be better scheduled. With the body being finished before the electrodes and/or the related conductors are pressed on the surface of the body, it is possible to better avoid any damages to the electrodes and Conductors during manufacturing.

With the manufacturing method comprising a conductor being formed from a metal wire, a non-conductive preform of the body of the mandrin being cast aground the metal wire and the distal end of the mandrin being formed in such a way that the end of the conductor is left bare at said distale end, it is possible to make the distal end of the mandarin difficult to deform since it is possible to keep the end of the metal wire and, at the same time, the end of the conductor securely at its place due to casting.

With the manufacturing method comprising an electrodes being manufactured in connection with said end of the conductor, it is possible to avoid the use of a separate conductor, whereby it is possible to save material and avoid the difficult connection of conductors.

With the manufacturing method comprising a foil being wrapped and fixed cylindrically around the body of the mandrin, a three-dimensional electrode pattern can be implemented, however, enabling said foil to be manufactured largely as a plane structure, whereby the production is made easier.

With the manufacturing method comprising conductors and/or functional electrode patterns being manufactured on both sides of the foil, it is possible to achieve an electrode pattern design closer to an optimum design since the space requirements of the conductors are thus utilized, the conductors being located on the other side of the foil.

### List of drawings

The invention is gone through in more detail in the following in the light of the exemplary embodiments shown in the attached drawings. Of these drawings:
- FIG 1A: is a schematic representation of a distal end of a mandrin and a needle of the arrangement according to the invention;
- FIG 18: is a schematic representation of the side and sectional view of the mandrin of the arrangement according to FIG 1A;

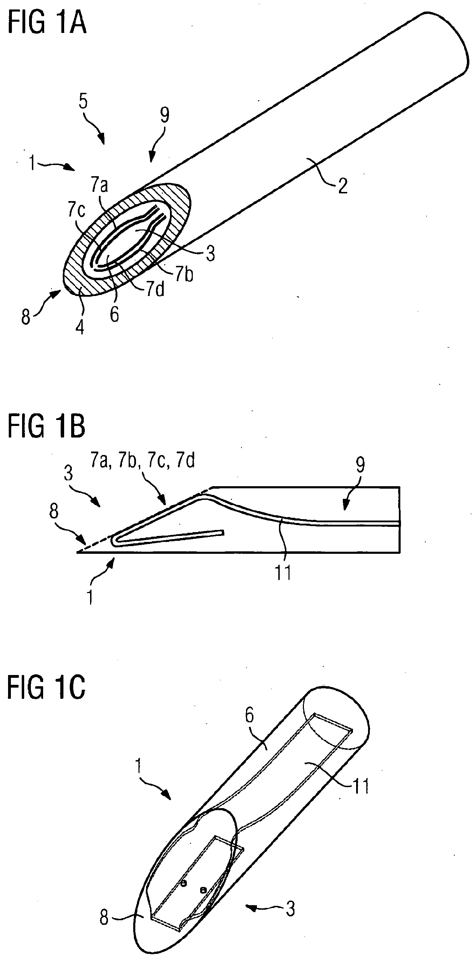

- FIG 1C: is a schematic representation of the interior parts of the mandarin of the arrangement according to FIG 1A;

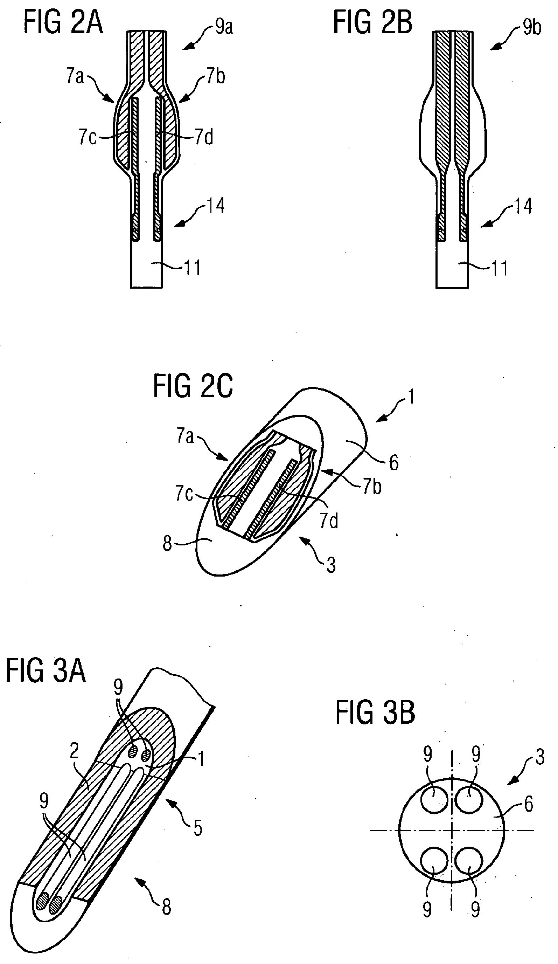

- FIG 2A: is a schematic representation of an electrode solution from the first direction according to a second embodiment of the invention;
- FIG 2B: is a schematic representation of an electrode solution from the other or opposite direction with reference to the first direction according to FIG 2A;
- FIG 2C: is a schematic representation of an electrode solution accommodated in the mandrin according to FIG 2A;
- FIG 3A: is a schematic representation of the distal end of a mandrin and a needle of the arrangement according to a third embodiment of the invention;
- FIG 3B: is a schematic representation of the distal end of the mandarin according to FIG 3A, in an axial view;

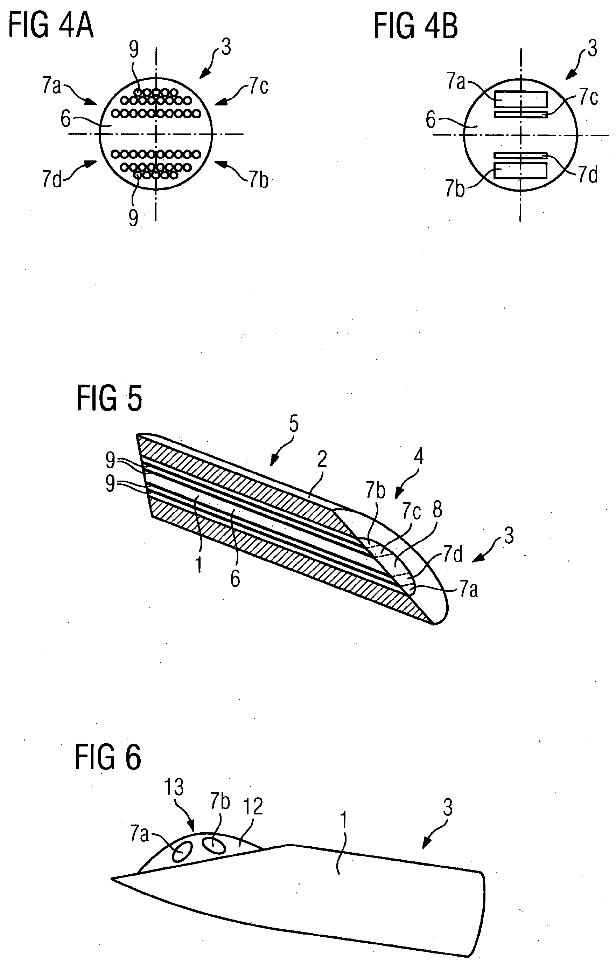

- FIG 4A and 4B: are schematic representations of the distal ends of mandrins according to a fourth and fifth embodiment of the invention, in an axial view;
- FIG 5: is a schematic representation of a sixth distal end of the mandrin and the needle belonging to the arrangement according to the invention, in a sectional view;
- FIG 6: is a schematic representation of a seventh distale end of the mandrin belonging to the arrangement according to the invention, in a side view;

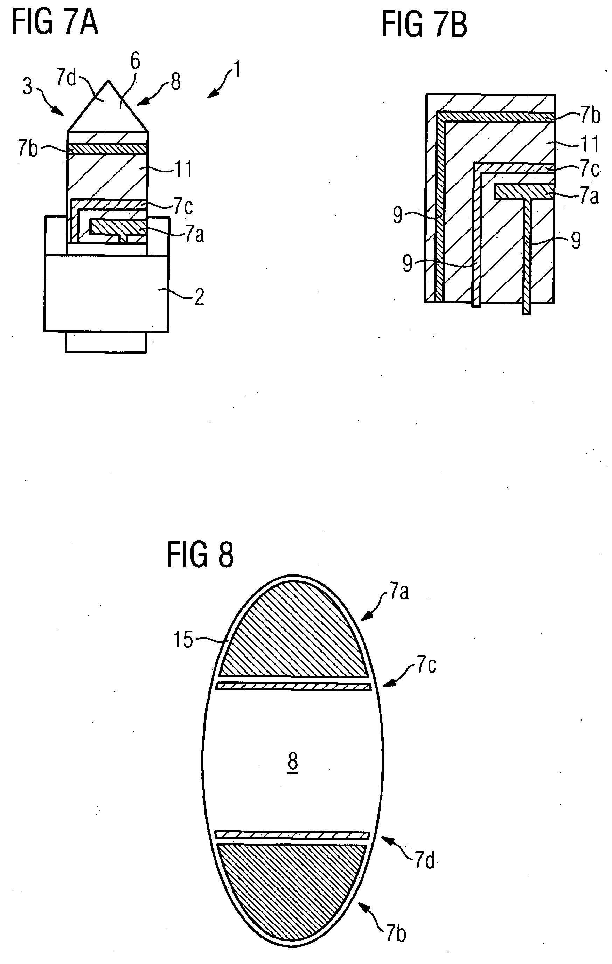

- FIG 7A: is a schematic representation of an eighth distale end of the mandrin belonging to the arrangement according to the invention, accommodated in a needle;
- FIG 78: is a schematic representation of an intermediate product of the manufacturing method of the mandrin according to FIG 3A;
- FIG 8: is a representation of an electrode pattern of the arrangement according to the invention;

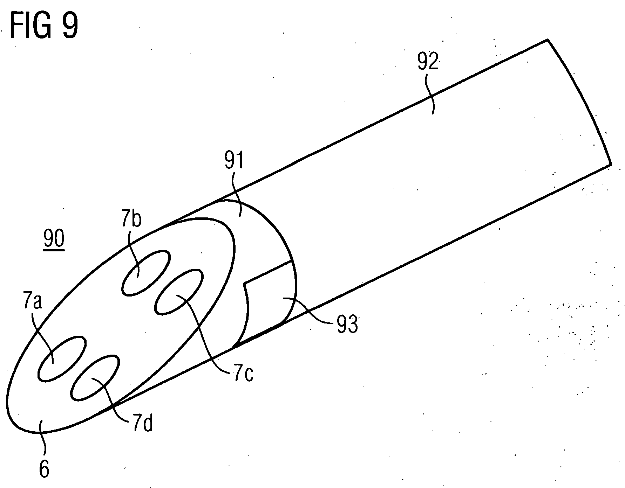

- FIG 9: is a representation of a mandrin formed by using a separate point part;

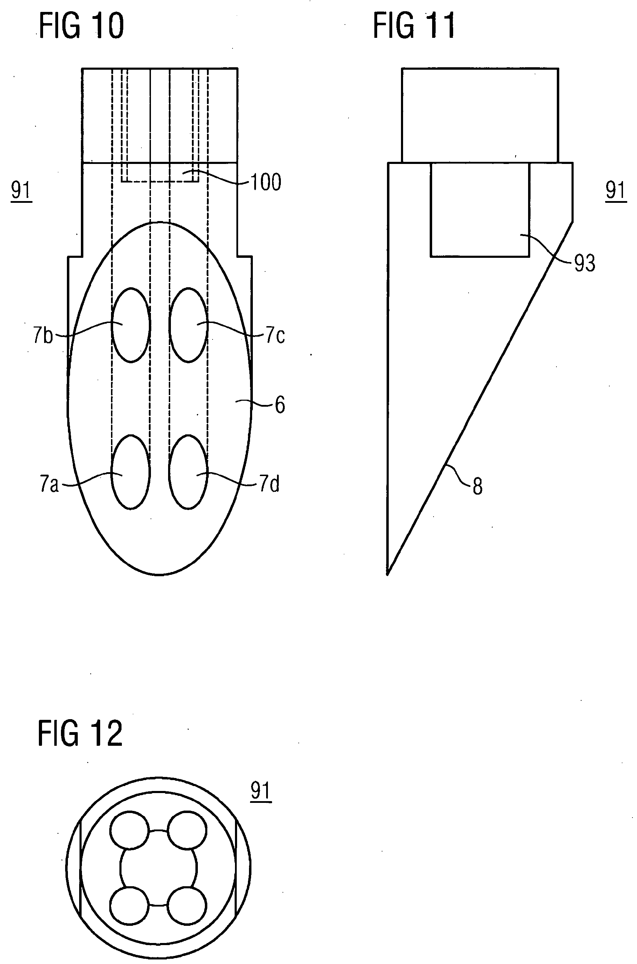

- FIG 10: is a representation of the point part of FIG 9, in a top view;
- FIG 11: is a representation of the point part of FIG 9, in a side view;
- FIG 12: is a representation of the point part of FIG 9, in a front view;

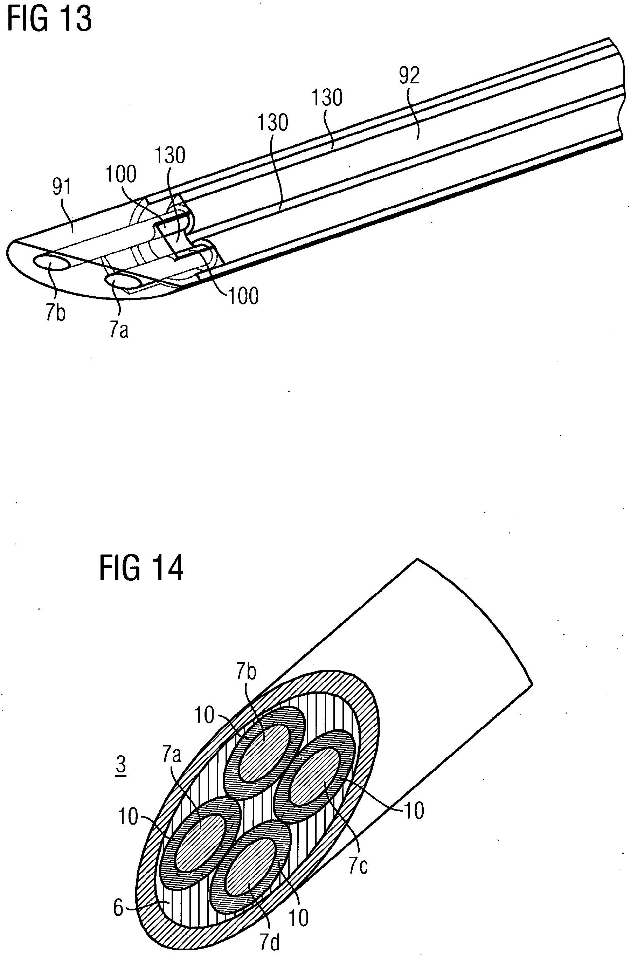

- FIG 13: is an illustration of the connection of a separate point part to form a mandrin;
- FIG 14: is an illustration of the mandrin, wherein four electrodes are formed by cutting metal wires, whereby the metal wires are at the same time useable as conductors from the electrode toward the processing unit;

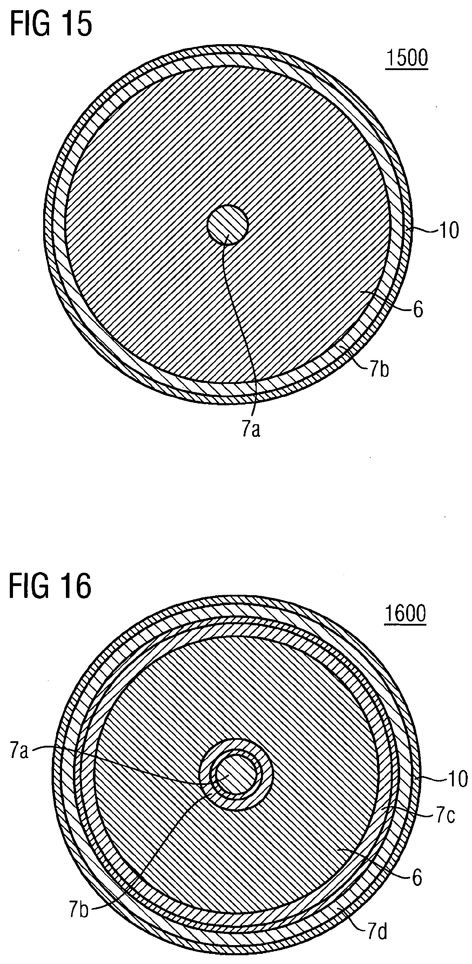

- FIG 15: shows the first coaxial electrode pattern; and
- FIG 16: shows the second coaxial electrode pattern.
For the sake of clarity, the embodiments represented in all Figures are shown in simplified mode. Similar parts are marked with identical reference numbers in all Figures.

### Detailed description of the invention

Shown in FIG. 1A is a schematic representation of a distal end of the mandrin and the needle of the arrangement according to the invention, shown in FIG 1B is a schematic representation of the same mandarin, shown in a side view and in a sectional view, and in FIG 1C a schematic representation of its interior parts is shown. The term "mandrin" above and in the following means the part to be inserted into a needle or a catheter and almost reaching the distal end at the distal end of the needle or the catheter, or even beyond it, and/or closing the distal end of the needle or the catheter. Sometimes the English term "stylus" or "mandarin" is used for a part like this in medical context.

The arrangement comprises mandrin 1 and hollow needle 2 in longitudinal channel 5 of which mandrin 1 is accommodated. Mandrin 1 is mobile in channel 5 and can be removed from there through the proximal end of channel 5. The proximal end has not been represented separately in FIG 1A to 1C.

Mandrin 1 comprises distal point 3 of mardrin and, correspondingly, needle has distal point 4 of needle. The distal point is the end of the mandrin and the needle that pentrates the tissue.

Needle 2 can be arranged as a part of a hypodermic syringe also comprising, apart, from needle 2, a container in a manner known as such, wherein the substance to be injected is accommodated, and devices by means of which the substance to be injected is forced through the longitudinal channel 5 of the needle into the organism after removing the mandrin 1 front the channel 5.

The substance to be injected can be a liquid or gaseous substance known as such.

It should be noted in this connection that in some further/alternative embodiments, the arrangement may comprise equipment for removing substances from the organism or for taking specimens from tissues. Furthermore, in some third embodiments, the arrangement may comprise equipment wherein a catheter is introduced into the body through a hollow needle enabling its use for conveying liquids or gases to tissues or for removing substances or specimens from the organism after removing the needle. Such known interventions take place in connection with urinary bladder, spinal column and blood vessels, among others.

Needle 2 can be a flexible catheter which can be bent according to the canals in the organism, or fundamentally inflexible and rigid, for example a hypodermic syringe. The flexural rigidity of mandrin 1 may vary from less than that of the needle 2 to more than that.

The distal end 3 of than mandrin comprises the bevel 8 forming the sharp point of the mandrin 1. The edges of the bevel 8 can have an incising effect in soft tissue. The cross section of the mandarin 1 being circular, the cross section of the bevel is oval.

The distal end 3 of the mandrin is of closed design as is preferably the complete body 6 of the mandrin. Among other things, this enables to obtain the advantage of the cross section of the distale end 3 of the mandrin being completely useable as a surface on which the electrodes can be arranged. Moreover, the closed design is as rigid as possible with respect to its diameter whereby the diameter of the mandarin 1 can be reduced without impairing its manageability. Due to the small diameter, the mandarin 1 penetrating the tissue causes very little damage to the tissue; moreover, the diameter of the needle can be reduced.

In the embodiment represented in FIG 1A to 1C, the distal end 3 of the mandrin only comprises one bevel 8. The distal end 3 of the mandril can nevertheless also be designed differently, for example in the form of a circular cone or as a surface formed by two or several intersecting bevels.

The bevels 8 do not necessarily have to be planar but may have convex and/or concave forms. However, the distal end 3 of the mandrin preferably has a form of which the cross section somehow converges, contributing to the penetration of the mandrin 1 into the tissues. For example, the distal end 3 of the mandrin can be designed according to the bevelled surface of the distal, incising end of needle 2.

Body 6 of the mandarin is manufactured of at least essentially non-conductive polymeric material, such as polypropylene PP or other polyolefin material. The body 6 can be manufactured for example by die-casting.

The four electrodes 7a to 7d intended for measuring bioimpedance are accommodated on bevel 8, forming a functional electrode pattern. Conductors 9 connect electrodes 7a to 7d to a processing unit which is not represented in FIG 1 to 3. This kind of electrode arrangement makes a so-called four-point measurement possible. The advantage of a system containing four electrodes is a reduction of faulty potentials due to the electrode impendance and a good measuring accuracy.

The number of electrodes in a functional electrode pattern can be two, three, four, five or even greater. All electrodes required for measuring bioimpedance are accommodated in mandrin 1. When the distal end 4 of the needle is at the right position within the organism, mandrin 1 is removed from needle 2 and the actual operation to be carried out by means of needle 2 can start.

In this case, the electrodes are accommodated to form two nesting circles. The outermost electrodes 7a and 7b are current electrodes and the innermost electrodes 7c and 7d are voltage electrodes. This kind of arrangement of current and voltage electrodes can be less sensitive to interferences than the opposite arrangement. The opposite arrangement is nevertheless also possible.

"Functional electrodes pattern" in this description means a pattern formed by electrodes and taking into account the characteristic features of bioimpedance measurement, possibly characterized by one or several of the four features listed bellow:
1. In an electrode pattern to be used in a four-point measurement, the current electrode and the corresponding electrode measuring the voltage difference are as close to each other as is allowed by the manufacturing technique. However, there is no galvanic contact between said electrodes.
2. The mutual distance between the electrodes measuring the voltage difference is optimized. For example, in four-point measurement this may mean that said mutual distance is greater than the distance between the pairs of current - voltage electrodes mentioned at item 1.
3. The electrodes do not reach the edge of the distal end but there is a non-conductive zone at the edge essentially isolating the electrodes from the needle 2.
4. The surface area of the electrodes is optimized in relation to the surface area available. The surface area available is limited by the dimensions of the needle 2 and the mandrin 1, any interferences due to the electrically conductive needle 2 and due to measurement focusing,

it should be mentioned as an example that a functional electrodes pattern accommodated in distal end 3, formed by one bevel 8, covers preferably at least 20 %, most preferably 25 to 50 %, of the bevel 8, and that the electrodes measuring the voltage are distinctly smaller than the current electrodes.

For example, it is very advantageous for carrying out a tetrapolar measurement of impedance to have an electrode pattern wherein the electrodes. 7a to 7d are arranged in parallel at the distal end 3 of the mandrin so that the distance between the current electrodes 7a and 7b and accordingly, the distance between the adjacent voltage electrodes 7c and 7d is as small as possible, and that the distance between the voltage electrodes 7c and 7d is as large as possible. The minimum possible distance between the current electrode and the voltage electrode minimizes the range of negative sensitivity. The maximum possible distance between the voltage electrodes maximizes the potential difference between them.

One method of manufacturing the mandrin 1 is to form required conductors 9 and electrodes 7a to 7d on a thin non-conductive foil 11, for example on polymeric foil, said foil being preferably in planar form during said forming. The polymeric foil can be, for example, a polyamide foil having a thickness of 18 to 50 µm. Conductors 9 and electrodes 7a to 7d can be manufactured by using a printing or a pressing method. In the printing method, for example, an ink-jet printer or an aerosol-jet printer can be used, the output being conductive ink. As pressing method, for example, tampon printing can be used.

Instead of the pointing or pressing method, or combined with them, metallized foil 11 can be used with conductors 9 and electrodes 7a to 7d being etched into it. Conductors 9 and electrodes 7a to 7d can be manufactured either on one side or on both sides of foil 11. Foil 11 forms a non-conductive substrate for electrically conductive patterns.

Said foil 11 can be cast as a part of mandrin 1 by using for example the die-casting method. Foil 11 can be accommodated in the die as an insert and the polymeric material can subsequently be cast in the die, whereby the foil 11 sticks on the molten polymeric mass. The casting can be done in one or several portions. Electrodes 7a to 7d are partly left on the surface at the distal end 3 of the mandrin but the conductors remain inside the body 6 of the mandrin. The connections to the measuring device can be implemented in modes known as such, or the measuring device can be integrated, at least partly, into the mandrin 1.

In one embodiment, the polymeric foil with a planar pattern is fixed on the outer surface of the body 6 of the mandrin by means of glue, by heating it, in other words, partly by melting, or by other corresponding means. The conductors 9 can thereby be accommodated between body 6 and the polymeric foil or, alternatively, they are left on the outer surface of the polymeric foil. The conductors 9 can be protected by covering them with a projective coating that is not electrically conductive and that can be manufactured for example by die-casting or by attaching a polymeric layer on conductors 9 which can be, for example, a parylene foil, polymethyl methacrylate (PMMA) or other polymeric material.

One advantage of manufacturing methods based on the foils 11, for example polyamide foils, lies in that electronic components, such as piezoelectric oscillators or amplifiers, can be integrated into the foil in addition to the conductors and/or the electrodes. A piezoelectric oscillator can be used as an ultrasonic transmitter and/or receiver, or it can be used to make the mandrin 1 oscillate in such a way that it and/or the needle 2 become visible on a dobbler ultrasound device. Other advantages of the foil 11 are its high adhesion to the body 6 of the mandrin, as well as the simplicity of manufacturing due to the fact that most stages of manufacturing, such as manufacturing of foil, combining metal with plastic and patterning, can be implemented in a two-dimensional plane. In one embodiment, foil 11 is a tube resembling a stocking with the conductors and the electrodes being formed on its surface in a manner previously described. For example, foil 11 can be a polyamide foil mentioned above. Body 6 of the mandrin can be of conductive maternal, such as steel or carbon fibre, and act as an electrode, or, alternatively, it can be of non-conductive material.

FIG 2A is a schematic representation of an electrode solution according to a further embodiment of the invention from the first direction, FIG 2B from the other, opposite direction with respect to the first direction, and FIG 2C accommodated in the mandrin.

Conductors 9a and 9b and the electrodes 7a to 7d have been accommodated on both sides of foil 11 in such a way that all electrodes 7a to 7d and conductors 9a of the first two electrodes 7a and 7b are arranged on the first surface of the foil, represented in FIG 2A. Conductors 9b of third and fourth electrode 7c and 7d are accommodated on the other surface of the foil 11, illustrated in FIG 2B. Third and fourth electrode 7c and 7d are connected to their respective conductors 9b by using electric bushings 14 reaching through the foil 11.

This has the advantage that the conductors may be made broader, which is of advantage in view of manufacturing easiness, reliability and electric functioning. It is also possible to connect several superimposed foils, preferably patterned only on one side, for example by laminating them. Furthermore, it is possible to increase the size of the electrodes 7a to 7d and the mutual distance of the electrodes in different potentials, which is beneficial to the accuracy of measurement.

Another manner of manufacturing the mandrin 1 is to manufactured body 6 for example by die-casting, after which electrodes 7a to 7d and/or the related conductive patterns or conductors 9 can be printed or pressed on body 6. The conductors can be coated in manners already mentioned above.

A third manner of manufacturing the mandrin 1 is to cast or press a preform of the body of the mandrin 1 in such a way that a selected number of metal wires 9, mutually positioned and designed in a suitable manner, is left inside the casting. A solution like this is schematically represented in FIG 3A and 3B. Each one of the conductors 9 on the left-hand side of FIG 3A and 3B can be connected to voltage electrodes or current electrodes and, accordingly, the ones on the right-hand side to current electrodes or voltage electrodes. Alternatively, the conductor at the top left, for example, is connected to a voltage electrode and the one at the bottom left to a current electrode, whereby the conductor 9 at the top right can be connected to a current electrodes and the conductor 9 at the bottom right to a voltage electrode.

The distance between conductors 9 can be managed for instance by using conductors provided with a polymer coating. Casting can be realized for example by die-casting and pressing by extrusion moulding, i.e. extruding or pultrusion. The distal end 3 of the mandrin is manufactured by cutting or shaping the preform in another manner.

The cross section of conductor 9 can be circular, rectangular or any other shape appropriate with respect to the electric measurement operation.

Shown in FIG 4A and 4B are schematic representations of distal end of a mandrin according to a fourth and fifth embodiment of the invention, in an axial view. The sectional areas of conductors 9 at the distal end 3 of the mandrin can thus form the electrodes 7a to 7d as such, as shown in FIG 7B. Furthermore, electrodes 7a to 7d can be formed by a functional pattern formed by two or several ends of conductor 9, as shown in FIG 7A.

The outer current electrodes 7a and 7d have a greater surface area than the inner electrodes 7b and 7c measuring the voltage difference. The greater surface area of the current electrodes 7a and 7d aims at keeping the current density at a suitable level and from increasing too much.

Shown in FIG 5 is a schematic representation of a sixth distal end of the mandrin and the needle belonging to the arrangement, shown in cross section, of an arrangement according to the invention. Conductors 9 are accommodated in body 6 of the mandrin, with the other end of said conductors reaching the distal end 3 of the mandarin. The conductors 9 may have been formed, for example, from metal wires in the manner already described above. Electrodes 7a to 7d can be manufactured by means of printing or pressing the functional electrode patterns on the sectional surface of the conductors 9 with the methods presented above - said sectional surface being formed at the distal end 3 of the mandrin - in such a way that electrodes. 7a to 7d establish an electric contact with the corresponding conductors. The functional pattern of the electrodes 7a to 7d can thus differ quite essentially from the shape of the sectional surface of the conductor.

Shown in FIG 6 is a schematic representation of a seventh distal end of the mandrin belonging to the arrangement according to the invention, in a side view. In this case, the distal end 3 of the mandrin consists of the bevel 8 having a bulge, i.e. ridge 12. electrodes 7a and 7b are accommodated in ridge 12. In addition, electrodes may be accommodated on bevel 8.

The ridge 12 rises from bevel 8 by a distance and increases the surface area of the distal end 3, which enables to accommodate several electrodes and/or larger electrodes at said end. A large electrode improves the quality of measurement.

The outermost part of the ridge 12 comprises an incising edge 13 capable of incising at least soft tissue. Moreover, the edge of bevel 8 can also comprise an incising edge.

The ridge 12 can be of the same structure as the body of the mandrin, in other words, it can be integrated into said body, or it can be a part manufactured separately and attached to its position at the distal and 3 of the mandrin.

It should be noted that in all embodiments of the arrangement, conductors 9 can be connected to a measuring device through the proximal end of the mandrin. The proximal end and the measuring device are not shown in FIG 6. In one embodiment, the wires leading to the measuring device are connected to the conductors 9 of the mandrin by means of soldering or by using connectors and by providing the connection with injection-moulding a holder that positions mandrin 1. In another embodiment, a part of the measuring device or even the complete measuring device is connected directly, i.e. integrated into the proximal end of the mandrin 1.

Shown in FIG. 7A is a schematic representation of a fourth distal end of the mandrin belonging to the arrangement according to the invention, in a side view and accommodated in a needle, and FIG 7B is a schematic representation of an intermediate product of the manufacturing method of said arrangement.

Body 6 of the mandrin comprises electrically conductive material which can be, for example, metal, such as steel. For example, the body 6 can be entirely of metal or it can be manufactured from an electrically conductive polymeric material or from a mixture comprising electrically conductive particle. The electrical conductivity of body 6 can also be achieve by using carbon fibre-doped polymeric material. The tip of the body 6 forms the circular cone-shaped bevel 8.

Wrapped on top or around the body 6 of the mandrin is foil 11 already described above, with electrode patterns of the three electrodes 7a to 7c formed on its surface along with the related conductors 9. Foil 11 can be attached to body 6, for axample, by heating, in order to produce chemical bonds between the foil and the body, or by using gline.

The body 6 of the mandrin is bare or, in other words, not covered by foil 11 at the utmost point of the distal end 3 of the mandrin so that it can be utilized as the fourth electrode 7d.

Mandrin 1 is accommodated in the channel of the needle 2 and can be moved from there distally and proximally with respect to the needle 2. The three electrodes 7a to 7c of the present embodiment are available for measuring the bioimpedance only when moved/transferred outside the needle 2. It is to be mentioned as an advantage of this embodiment that bioimpedance can only be measured when the mandrin 1, smaller in size to the needle 2 itself, has been pushed ahead of it, whereby damages to the penetrated tissues can be minimized. In addition, it is possible to anticipate the location of the distal end of the needle 2 within the organism very accurately.

The mandrin 1 can be moved with respect to the needle 2 in many different ways, for example, by means of a thread or a spring solution.

Shown in FIG 8 is a representation of an electrode pattern of an arrangement according to the invention. The electrode pattern comprises two pairs of electrodes, each with a current electrode 7a and 7b and an electrode 7c and 7d for measuring the voltage difference. In a pair of electrodes, the distance between the electrodes is preferably 0.1 mm or less. There is a non-conductive zone 15 between the electrodes 7a to 7d and the edge of the bevel 8.

The electrode pattern is accommodated on a bevel 8. Electrodes 7a to 7d cover preferably at least 20 %, most preferably 25 to 50 % of the surface area available, in this case of the surface area of bevel 8. The contact of the electrodes to the tissue is then good. In the embodiment represented in FIG 8, the surface area of the electrodes 7a to 7d is approximately 42 % of the surface area of the bevel which is a percentage that has been found very advantageous.

The arrangement and the method according to the invention have many advantages, for example:
a) Information is acquired on the properties of the tissue. It is possible to deduce from this information the tissue in which the needle is located.
b) With the conductors, any other electronics belonging to the solution and the insulating layers being implemented within the mandrin, the outer diameter of the needle does not increase, which is of advantage Medically, among other aspects, with regard to minimising pain, risk of inflamination and hemorrhage.
c) An external needle protects the conductors and the electrodes located inside the mandrin.
d) With the needle at its right position, the mandrin can be removed, which improves the useability of the needle. For example, any wires or electronics are afterwards not in the way of operating the needle.

FIG 9 shows a perspective view of the mandrin 90, formed by using a separate point part 91. FIG 10 shows a top view of the point part 91, FIG 11 a side view and FIG 12 a front view of it.

The point part 91 comprises electrodes 7a, 7b, 7c and 7d or, alternatively, holes for the electrode wires. Moreover, the point part 91 may also comprise a hollow 93 or a guiding surface which can be used for orientation of the point part 91 during manufacturing of the point part 91. The electrodes 7a, 7b, 7c and 7d may reach up to the hollow 93.

To form mandrin 90, point part 91 is connected to the conductor part as illustrated in FIG 13. FIG 13 illustrates the mandrin 90, shown in sectional view.

Each electrode wire 100 located in its own polyamide tube 92 or most commonly coated with insulating layer 10 is introduced through the end of the point part 91. When the galvanic contact has been established between the electrodes 7a, 7b, 7c and 7d and the electrode wires 100, the structure is joined together with glue 130 or with a hardening casting compound. Instead of the electrodes located in point part 91, the cut and optionally coated ends of the electrode wires 100 may act as electrodes.

FIG 14 illustrates mandrin 3 with four electrodes 7a, 7b, 7c and 7d formed by breaking off or cutting metal wires, whereby the metal wires are at the same time useable as conductors from the electrode toward the processing unit. The ends of the metal wires that have been broken off or cut can be coated as required.

The body of mandrin 3 shown in FIG 14 is made of filling material, preferably of silicone for medical use or, for example, of a polymeric material suitable for the same purpose, and preferably reaches into the mandrin 3 by a distance (for example, by a few millimetres). The body 6 is covered with a polyamide tube or, more generally, with an insulating layer 10 which continues beyond the body 6 in the longitudinal direction of the electrodes 7a, 7b, 7c and 7d.

The electrode pattern of the mandrins 1500 and 1600 can also be implemented coaxially as is shown in FIG 15 and 16. For the sake of clarity, the needle 2 has been omitted in the figures.

It is obvious to the skilled person that, along with the technical progress, the basic idea of the invention can be implemented in many ways . The invention and its embodiments are thus not limited to the examples described above but they May vary within the contents of patent claims and their legal equivalents.

In particular even though the embodiments of the mandarins and the point parts according to the invention described above are mainly presented by means of a tetrapolar electrode configuration, mandrins 1 and 90 and distal end 3 of the mandrin as well as point part 91 can be of bipolar design according to the same principles, in other words, by means of only two electrodes.

In addition to this or instead of this, needle 2 or the shell of the mandrins 1, 90, 1500 and 1600 or a part of it can be earthed or put into a known potential. The error caused by needle 2 can thus be further minimised.

### List of reference numbers used

- 1, 90, 1500, 1600: mandrin
- 2: needle
- 3: distal end of the mandrin
- 4: distal end of the needle
- 5: channel of the needle
- 6: body of the mandrin
- 7a to 7d: electrode
- 8: bevel
- 9, 9a, 9b: conductor
- 10: insulating layer
- 11: foil
- 12: ridge
- 13: incising edge
- 14: electric bushing
- 15: non-conductive zone
- 91: point part
- 92: polyamide tube
- 93: guiding surface
- 100: electrode wire
- 130: glue

## Claims

1. Arrangement for defining a location within an organism, said arrangement comprising:
- a hollow needle (2), comprising a longitudinal channel (5) and a distal end (4);
- a mandrin (1, 90, 1500, 1600), accommodated in the longitudinal channel (5) in mobile manner in relation to it and removable from said channel (5) and having a distal end (3) of closed design, comprising an electrode pattern including at least two electrodes (7a, 7b, 7c, 7d); as well as
- a processing unit, configured to monitor the advancement of the distal end (4) only on the basis of a measurement signal of bioimpedance received from the electrodes (7a, 7b, 7c, 7d) of the electrode pattern.

2. Arrangement according to claim 1, wherein the functional electrode pattern comprises two voltage electrodes and two current electrodes.

3. Arrangement according to claim 2, wherein the electrodes pattern comprises two pairs of electrodes, each having a current electrode and a voltage electrode, the distance between the pairs of electrodes being greater than the distance between electrodes belonging to one and the same pair of electrodes, and wherein the electrodes (7a to 7d) cover at least 20 % of the surface area available at the distal end (3) of the mandrin.

4. Arrangement according to any one of the above claims, wherein the distal end (3) of the mandrin (1, 90, 1500, 1600) comprises a sharp point.

5. Arrangement according to claim 4, wherein the sharp point is composed of a bevel (8) edge and the electrodes (7a, 7b, 7c, 7d) are accommodated on said bevel (8).

6. Arrangement according to claim 5, wherein the bevel (8) comprises a ridge (12) accommodating at least one electrode (7a, 7b, 7c, 7d).

7. Arrangement according to any one of the above claims, wherein the needle (2) is a flexible catheter which can be bent according to a canal in the organism.

8. Arrangement according to any one of the above claims, wherein at least some of the electrodes (7a, 7b, 7c, 7d) are formed by using at least one metal wire (100) by cutting or by breaking it off and optionally also by coating its end that is to become an electrode (7a, 7b, 7c, 7d), whereby the wire (100) is at the same time useable as a conductor from the electrode (7a, 7b, 7c, 7d) toward the processing unit.

9. Arrangement according to any one of the above claims, wherein at least some of the electrodes (7a, 7b, 7c, 7d) of the electrode pattern have been arranged coaxially.

10. Arrangement according to any one of the above claims, wherein the body (6) of the mandrin (1, 90, 1500, 1600) is exposed in such a way that it is useable as one of the electrodes.

11. Method for manufacturing a mandrin (11) to be accommodated in a needle, **characterized in that** in the method an electrode pattern is accommodated at the closed distal end (3) of the mandrin and contains at least two electrodes (7a, 7b, 7c, 7d) that are suitable for measuring the bioimpedance of tissues and that are useable for generating a measurement signal of bioimpedance within an organism on the basis of which the advancement of the distal end (4) can be monitored by means of a processing unit.

12. Method according to claim 11, wherein an electrode (7a, 7b, 7c, 7d) is produced by printing or pressing a conductive functional electrode pattern on a non-conductive foil (11), a conductor (9) is accommodated in said electrode pattern and a mandrin (1, 90, 1500, 1600) is formed by arranging the functional electrode pattern at its distal end (3) and by accommodating the conductors (9) in the body (6) of the mandrin.

13. Method according to claim 12, wherein said conductor (9) is printed or pressed on said foil (11), the foil (11) is bent and the Body (6) of the mandrin is cast around the foil (11) so that the functional electrode pattern is left on the surface of the distal end (3) of the mandrin and the conductors (9) is left inside the body (6) of the mandrin.

14. Method according to claim 12 or 13, wherein the conductive functional electrode pattern is printed or pressed on said substrate being in the form of plane surface.

15. method according to claim 11, wherein the body (6) of the mandrin is manufactured and the electrodes (7a - 7d) and/or the related conductors (9) are printed or pressed on the surface of the body (6).

16. Method according to claim 11, wherein a conductor (6) is formed from metal wire, a non-conductive preform of the body (6) of the mandrin is cast around the metal wire and the distal end (3) of the mandrin is formed if such a way that the end of the conductor (9) is left bare at said distal end (3).

17. Method according to claim 16, wherein an electrode (7a, 7b, 7c, 7d) is made in connection with said conductor (9).

18. method according to claim 12, wherein a foil (11) is wrapped and fixed cylindrically around the body (6) of the mandrin.

19. method according to any one of the claims 12 to 14, wherein conductors and/or functional electrode patterns are made on both sides of the foil (11).

## Patentansprüche

1. Anordnung zur Bestimmung einer Stelle innerhalb eines Organismus, die folgendes umfasst:
- eine Hohlnadel (2), die einen Längskanal (5) und ein distales Ende (4) umfasst;
- einen Mandrin (1, 90, 1500, 1600), der im Längskanal (5) auf bewegliche Weise im Verhältnis zu diesem untergebracht ist und aus dem Kanal (5) entfernbar ist und ein distales Ende (3) geschlossener Konstruktion aufweist, das ein Elektrodenmuster mit mindestens zwei Elektroden (7a, 7b, 7c, 7d) umfasst; sowie
- eine Prozessoreinheit, die zur Überwachung des Vordringens des distalen Endes (4) nur auf Basis eines Bioimpedanz-Messsignals konfiguriert ist, das sie von den Elektroden (7a, 7b, 7c, 7d) des Elektrodenmusters erhält.

2. Anordnung nach Anspruch 1, in der das funktionelle Elektrodenmuster zwei Spannungselektroden und zwei Stromelektroden umfasst.

3. Anordnung nach Anspruch 2, in der das Elektrodenmuster zwei Elektrodenpaare umfasst, von denen jedes eine Stromelektrode und eine Spannungselektrode aufweist, wobei der Abstand zwischen den Elektrodenpaaren größer ist, als der Abstand zwischen Elektroden, die zu ein und dem selben Elektrodenpaar gehören, und in der die Elektroden (7a bis 7d) mindestens 20% des am distalen Ende (3) des Mandrins vorhandenen Oberflächenbereichs abdeckt.

4. Anordnung nach einem der vorhergehenden Ansprüche, in der das distale Ende (3) des Mandrins (1, 90, 1500, 1600) eine scharfe Spitze umfasst.

5. Anordnung nach Anspruch 4, in der die scharfe Spitze aus einer schrägen (8) Kante besteht und die Elektroden (7a, 7b, 7c, 7d) auf der Schräge (8) untergebracht sind.

6. Anordnung nach Anspruch 5, in der die Schräge (8) einen Rücken (12) umfasst, der mindestens eine Elektrode (7a, 7b, 7c, 7d) fasst.

7. Anordnung nach einem der vorhergehenden Ansprüche, in der die Nadel (2) ein flexibler Katheter ist, der nach einem Kanal im Organismus biegbar ist.

8. Anordnung nach einem der vorhergehenden Ansprüche, in der mindestens einige der Elektroden (7a, 7b, 7c, 7d) durch Verwendung mindestens eines Metalldrahtes (100) durch Abschneiden oder Abbrechen desselben und wahlweise auch durch Beschichtung seines Endes gebildet sind, das zur Elektrode (7a, 7b, 7c, 7d) werden soll, wonach der Draht (100) gleichzeitig als Leiter von der Elektrode (7a, 7b, 7c, 7d) zur Prozessoreinheit verwendbar ist.

9. Anordnung nach einem der vorhergehenden Ansprüche, in der mindestens einige der Elektroden (7a, 7b, 7c, 7d) des Elektrodenmusters koaxial angeordnet sind.

10. Anordnung nach einem der vorhergehenden Ansprüche, in der der Körper (6) des Mandrins (1, 90, 1500, 1600) auf solch eine Weise freigelegt ist, dass er als eine der Elektroden verwendbar ist.

11. Verfahren zur Herstellung eines Mandrins (11) zur Unterbringung in einer Nadel, **dadurch gekennzeichnet, dass** in dem Verfahren ein Elektrodenmuster an dem geschlossenen distalen Ende (3) des Mandrins untergebracht ist und mindestens zwei Elektroden (7a, 7b, 7c, 7d) enthält, die sich zur Bioimpedanz-Messung von Geweben eignen und zur Erzeugung eines Bioimpedanz-Messsignals innerhalb eines Organismus verwendet werden können, auf Basis dessen das Vordringen des distalen Endes(4) durch eine Prozessoreinheit überwacht werden kann.

12. Verfahren nach Anspruch 11, in dem eine Elektrode (7a, 7b, 7c, 7d) durch Aufdrucken oder Aufpressen eines leitenden funktionellen Elektrodenmusters auf eine nichtleitende Folie (11) produziert wird, ein Leiter (9) im Elektrodenmuster untergebracht wird und ein Mandrin (1, 90, 1500, 1600) durch Anordnung des funktionellen Elektrodenmusters an seinem distalen Ende (3) und durch Unterbringen des Leiters (9) im Körper (6) des Mandrins gebildet wird.

13. Verfahren nach Anspruch 12, in dem der Leiter (9) auf die Folie (11) aufgedruckt oder aufgepresst wird, die Folie (11) gebogen ist und der Körper (6) des Mandrins um die Folie (11) herumgegossen wird, so dass das funktionelle Elektrodenmuster auf der Oberfläche des distalen Endes (3) des Mandrins verbleibt und der Leiter (9) innerhalb des Körpers (6) des Mandrins verbleibt.

14. Verfahren nach Anspruch 12 oder 13, in dem das leitende funktionelle Elektrodenmuster auf das Substrat, das die Form einer ebenen Oberfläche hat, aufgedruckt oder aufgepresst wird.

15. Verfahren nach Anspruch 11, in dem der Körper (6) des Mandrins hergestellt wird und die Elektroden (7a - 7d) und/oder die damit zusammenhängenden Leiter (9) auf die Oberfläche des Körpers (6) aufgedruckt oder aufgepresst werden.

16. Verfahren nach Anspruch 11, in dem ein Leiter (6) aus Metalldraht gebildet wird, ein nichtleitender Vorformling des Körpers (6) des Mandrins um den Metalldraht herum gegossen wird und das distale Ende (3) des Mandrins auf eine solche Weise geformt wird, dass das Ende des Leiters (9) am distalen Ende (3) frei bleibt.

17. Verfahren nach Anspruch 16, in dem eine Elektrode (7a, 7b, 7c, 7d) in Verbindung mit dem Leiter (9) gemacht wird.

18. Verfahren nach Anspruch 12, in dem eine Folie (11) zylindrisch um den Körper (6) des Mandrins herum gewickelt und befestigt wird.

19. Verfahren nach einem der Ansprüche 12 bis 14, in dem Leiter und/oder funktionelle Elektrodenmuster auf beide Seiten der Folie (11) gemacht werden.

## Revendications

1. Dispositif pour définir un site à l'intérieur d'un organisme, ledit dispositif comprenant :
- une aiguille creuse (2), comprenant un canal longitudinal (5) et une extrémité distale (4) ;
- un mandrin (1 90, 1500, 1600) logé dans le canal longitudinal (5) de manière mobile par rapport à ce dernier et pouvant être retiré dudit canal (5) et comportant une extrémité distale (3) de type fermé, et comprenant un ensemble d'électrodes comprenant au moins deux électrodes (7a, 7b, 7c, 7d) ; ainsi qu'une
- unité de traitement, conçue pour contrôler l'avancée de l'extrémité distale (4) uniquement sur la base d'un signal de mesure de bio-impédance reçu depuis les électrodes (7a, 7b, 7c, 7d) de l'ensemble d'électrodes.

2. Dispositif selon la revendication 1, dans lequel l'ensemble d'électrodes fonctionnelles comprend deux électrodes de tension et deux électrodes de courant.

3. Dispositif selon la revendication 2, dans lequel l'ensemble d'électrodes comprend deux paires d'électrodes chacune composée d'une électrode de courant et d'une électrode de tension, la distance séparant lesdites paires d'électrodes étant supérieure à la distance séparant les électrodes appartenant à la même paire d'électrodes, et dans lequel les électrodes {7a à 7d) couvrent au moins 20 % de la surface disponible à l'extrémité distale (3) du mandrin.

4. Dispositif selon l'une quelconque des revendications précédentes, dans lequel l'extrémité distale (3) du mandrin (1, 90, 1500, 1600) comprend une pointe acérée.

5. Dispositif selon la revendication 4, dans lequel la pointe acérée comprend un bord biseauté (8) et les électrodes (7a, 7b, 7c, 7d) sont logées dans ledit bord biseauté (8).

6. Dispositif selon la revendication 5, dans lequel le bord biseauté (8) comprend une arête (12) dans laquelle est logée au moins une électrode (7a, 7b, 7c, 7d).

7. Dispositif selon l'une quelconque des revendications précédentes, dans lequel l'aiguille (2) est un cathéter flexible pouvant être plié selon un canal de l'organisme.

8. Dispositif selon l'une quelconque des revendications précédentes, dans lequel l'une au moins des électrodes (7a, 7b, 7c, 7d) est formée à l'aide d'au moins un câble métallique (100) qui a été coupé ou cassé et dont l'extrémité amenée à devenir une électrode (7a, 7b, 7c, 7d) est éventuellement dotée d'un revêtement, ledit câble (100) pouvant également être utilisé comme conducteur depuis l'électrode (7a, 7b, 7c, 7d) vers l'unité de traitement.

9. Dispositif selon l'une quelconque des revendications précédentes, dans lequel l'une au moins des électrodes (7a, 7b, 7c, 7d) de l'ensemble d'électrodes est disposée de manière coaxiale.

10. Dispositif selon l'une quelconque des revendications précédentes, dans lequel le corps (6) du mandrin (1, 90, 1500, 1600) est disposé de telle sorte qu'il peut être utilisé comme l'une des électrodes.

11. Procédé de fabrication d'un mandrin (11) à loger dans une aiguille, **caractérisé en ce qu'**un ensemble d'électrodes est logé dans l'extrémité distale (3) de type fermé du mandrin et comprend au moins deux électrodes (7a, 7b, 7c, 7d) capables de mesurer la bio-impédance des tissus et pouvant être utilisées pour générer un signal de mesure de bio-impédance dans un organisme sur la base duquel l'avancée de l'extrémité distale (4) peut être contrôlée au moyen d'une unité de traitement.

12. Procédé de fabrication selon la revendication 11, dans lequel une électrode (7a, 7b, 7c, 7d) est produite par impression ou pression d'un ensemble d'électrodes fonctionnelles conductrices sur une feuille non-conductrice (11) et un conducteur (9) est logé dans ledit ensemble d'électrodes fonctionnelles et un mandrin (1, 90, 1500, 1600) est formé en disposant l'ensemble d'électrodes fonctionnelles à son extrémité distale (3) et en logeant le conducteur (9) dans le corps dudit mandrin.

13. Procédé de fabrication selon la revendication 12, dans lequel ledit conducteur (9) est imprimé ou pressé sur ladite feuille (11), la feuille (11) étant pliée et le corps (6) du mandrin étant moulé autour de ladite feuille (11) de sorte que l'ensemble d'électrodes fonctionnelles demeure à la surface de l'extrémité distale (3) du mandrin et le conducteur (9) demeure à l'intérieur du corps (6) du mandrin.

14. Procédé de fabrication selon la revendication 12 ou la revendication 13, dans lequel l'ensemble d'électrodes fonctionnelles conductrices est imprimé ou pressé sur un substrat ayant la forme d'une surface plane.

15. Procédé de fabrication selon la revendication 11, dans lequel le corps (6) du mandrin est fabriqué et les électrodes (7a - 7d) et/ou les conducteurs associés (9) sont imprimés ou pressés sur la surface dudit corps (6).

16. Procédé de fabrication selon la revendication 11, dans lequel un conducteur (6) est formé d'un câble métallique et une préforme non-conductrice du corps (6) du mandrin est modelée autour dudit câble métallique et l'extrémité distale (3) du mandrin est formée de sorte que l'extrémité du conducteur (9) est laissé nue à ladite extrémité distale (3).

17. Procédé de fabrication selon la revendication 16, dans lequel une électrode (7a, 7b, 7c, 7d) est fabriquée en relation avec ledit conducteur (9).

18. Procédé de fabrication selon la revendication 12, dans lequel une feuille (11) est enveloppée et fixée de manière cylindrique autour du corps (6) du mandrin.

19. Procédé de fabrication selon l'une quelconque des revendications 12 à 14, dans lequel les conducteurs et/ou les ensembles d'électrodes fonctionnelles sont réalisés sur les deux côtés de la feuille (11).
